# EUROPEAN PATENT SPECIFICATION

(11) **EP 3 725 271 B1**
(45) Date of publication and mention of the grant of the patent: **09.12.2026**
(21) Application number: 20161310.6
(22) Date of filing: 05.03.2020
(51) Int. Cl.: A61F 2/50, A61F 2/60, A61F 2/78, A61F 2/70, A61F 2/74, A61F 2/76

(54) **INFLATABLE LIMB PROSTHESIS**
AUFBLASBARE GLIEDMASSENPROTHESE
PROTHÈSE DE MEMBRE GONFLABLE

(30) Priority: 16.04.2019 IL 26607419
(43) Date of publication of application: 21.10.2020
(73) Proprietor: Y.D. Gapim Ltd, Rishon Lezion 756339 (IL); Pilosof, Israel, 7031709 Beer Yaakov (IL); Pilosof, Yehud, 7542141 Rishon Lezion (IL)
(72) Inventor: PILOSOF, Israel, 7031709 Beer Yaakov (IL); PILOSOF, Yehuda, 7542141 Rishon Lezion (IL); ARLINKSY, David, 3030011 Atlit (IL)
(74) Representative: Harrison IP Limited

(56) References cited:
- EP-A1- 2 327 378
- WO-A1-2008/098053
- WO-A1-2017/221019
- WO-A2-2013/049847
- GB-A- 698 150
- US-A1- 2016 228 266
- US-A1- 2018 020 973

## Description

### TECHNICAL FIELD

The present invention relates to the field of limb prostheses.

### BACKGROUND ART

In medicine, prosthesis is an artificial device that replaces a missing body part, which may be lost through trauma, disease, or congenital conditions. Prosthetics are intended to restore the normal functions of the missing body part.

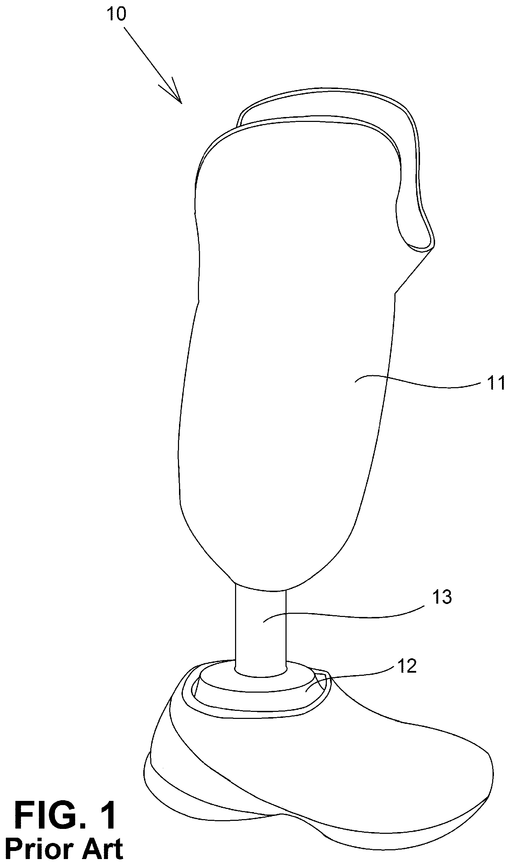

Fig. 1 schematically illustrates a foot prosthesis, according to the prior art.

The prosthesis comprises a socket 11, connected to a prosthetic foot 12 by a joint pipe 13.

The prosthesis comprises also a lining disposed inside the socket 11. The lining is not seen in this figure.

While the socket 11 is made of "hard" material, the lining is made of "soft" material, such as sponge and gel, in order to soften the contact of the user stump (the distal end of a limb left after amputation) with the socket 11.

**Fig. 2** schematically illustrates user using a foot prosthesis, according to the prior art.

In this figure is seen also a sock 15, worn on the stump 14. The sock adds softness to the contact of the stump with the prosthesis. The dashed line 14 illustrates the foot stump hidden behind the prosthesis socket.

**Fig. 3** schematically illustrates steps in mounting a prosthesis, according to the prior art.
In step (a) there is shown a bare stump 14;
In step (b) the user wears one or more socks 15 on the stump 14;
In step (c) the user wears a lining 16 on sock 15; and
In step (d) the user wears a socket 11 on the lining.

During the day, the stump may change its size, i.e., to shrink or to expand. As such, the prosthesis socket loses its correct fit to the user's stump. In order to overcome this obstacle, the user may wear during the day additional or less sock(s), an action which may be embarrassing and inconvenient.

US5405405A discloses a composite socket member for use with a prosthetic appliance for a residual limb which comprises an outer socket which defines an inner cavity generally conforming to the outer surface of a residual limb, an inner socket which defines an inner cavity and being adapted to receive the residual limb, with the inner socket conforming to the shape of the outer socket and when nested within the cavity of the outer socket defines an air space between the inner surface of the outer socket and the outer surface of the inner socket and an inflatable bladder being disposed between the inner surface of the outer socket and the outer surface of the inner socket. The inner socket contains at least one opening through its side wall at a preselected weight-bearing location whereby upon inflation of the bladder, pressure is applied by the bladder through the side wall opening against the preselected weight-bearing location of the residual limb to control the movement and rotation stability of the prosthetic appliance.

EP2327378A1 discloses a cap for the application of a limb prosthesis, in particular a prosthesis for a lower limb, comprises a dual membrane structure, with a first inner membrane and a second outer membrane, defining a sealed hollow space. The inner membrane defines a volume for seating the limb stump and the outer membrane defines a surface of contact and adhesion to the socket of the prosthesis. The presence of substantially inextensible elements makes the outer membrane deform radially outwards under the effect of the air under pressure into hollow space, forming a plurality of swellings between each pair of consecutive linear substantially inextensible elements.

All the methods described above have not yet provided satisfactory solutions to the problem of adjusting a prosthesis pressure on a limb stump.

It is an object of the present invention to provide a solution to the above-mentioned and other problems of the prior art.

Other objects and advantages of the invention will become apparent as the description proceeds.

### SUMMARY OF THE INVENTION

According to an aspect of the present invention, there is provided a limb prosthesis (10) comprising: means for changing an inner size of a socket (11) of said limb prosthesis (10), said means comprising: an inner inflatable lining (16) and a pump assembly (19) for inflating and deflating said inflatable lining (16), wherein said inner inflatable lining (16) comprises only longitudinal inflatable cells (20) adjoining each other,
characterized in that said means for changing an inner size of the socket (11) of said limb prosthesis (10) further comprises a remote control (17) in data communication with said pump assembly (19), said remote control (17) configured to instruct said pump assembly (19) to inflate or deflate said lining (16) to a desired air pressure;
and in that each inner inflatable cell (20) comprises an inwardly-facing surface having a convex curvature, each cell (20) being configured to be inflated to said desired air pressure, thereby generating a gap between each two adjoining cells (20) and a stump placed in said prosthesis.

The prosthesis may further comprise a pressure sensor and a controllable valve, for allowing inflating and deflating said lining (16) to a desired pressure.

The limb prosthesis may further comprise a circuitry adapted to retain constant pressure in the lining. This may free the controller to be in continuous or alternate contact with the pump assembly.

According to one embodiment of the invention, the remote control (17) is a smartphone. This allows the user to instruct the pump assembly (19) to instruct the prosthesis to change the pressure of the lining without attracting attention of nearby individuals, since nowadays smartphones are very common, and using them in public is a common behavior.

The data communication is preferably wireless; however, it can also be wired.

The limb prosthesis may further comprise means for manually deflating said lining, thereby allowing deflating the lining in the case of an operational fault.

According to one embodiment of the invention, the means for changing an inner size of a socket of the limb prosthesis by a remote control are pneumatic.

The reference numbers have been used to point out elements in the embodiments described and illustrated herein, in order to facilitate the understanding of the invention. They are meant to be merely illustrative, and not limiting. Also, the foregoing embodiments of the invention have been described and illustrated in conjunction with systems and methods thereof, which are meant to be merely illustrative, and not limiting.

### BRIEF DESCRIPTION OF DRAWINGS

Preferred embodiments, features, aspects and advantages of the present invention are described herein in conjunction with the following drawings:
**Fig. 1** schematically illustrates a foot prosthesis, according to the prior art.

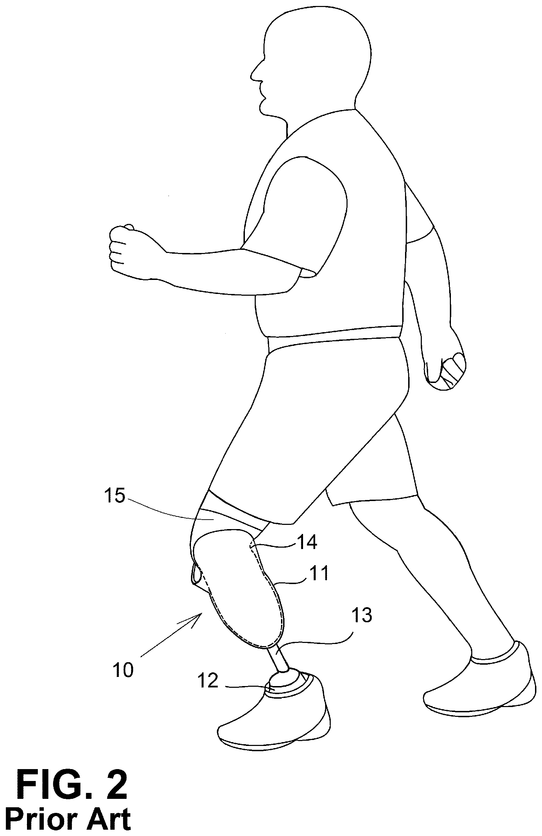

**Fig. 2** schematically illustrates user using a foot prosthesis, according to the prior art.

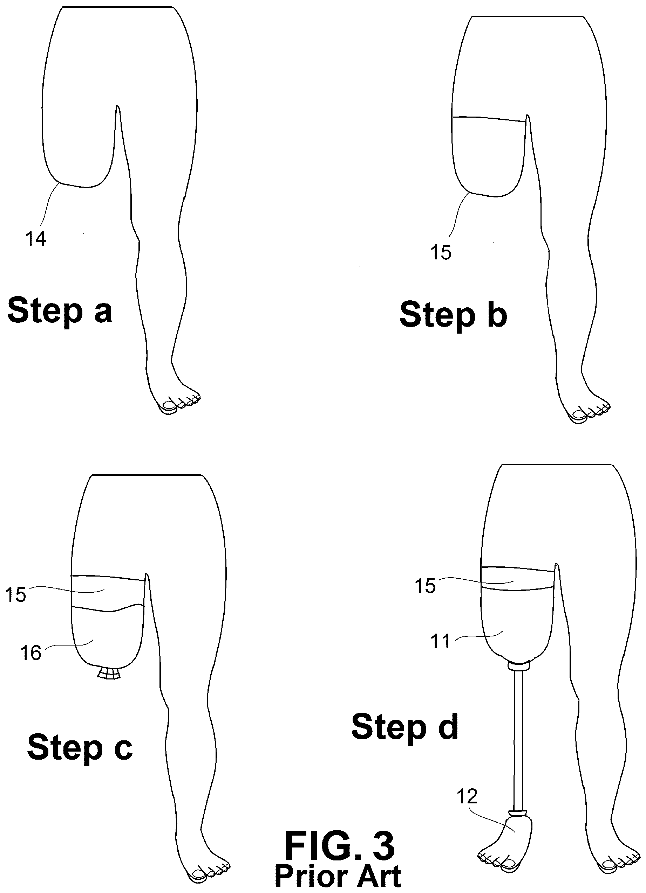

**Fig. 3** schematically illustrates steps in mounting a prosthesis, according to the prior art.

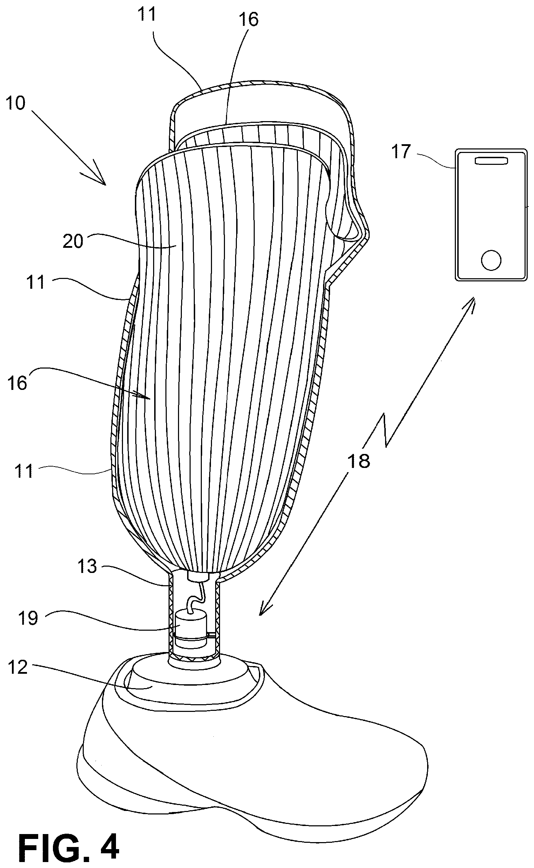

**Fig. 4** schematically illustrates a prosthesis, according to one embodiment of the invention.

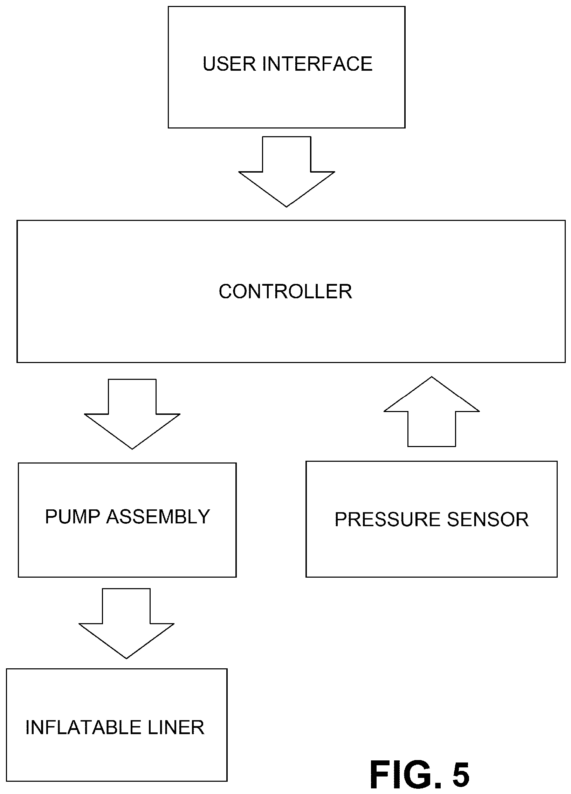

**Fig. 5** is a block diagram schematically illustrating the dataflow in a prosthesis, according to one embodiment of the invention.

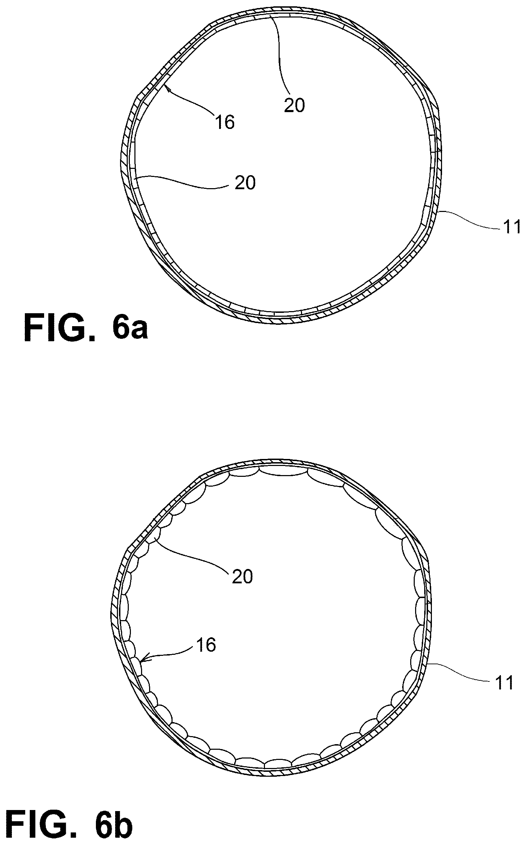

**Fig. 6a** is a sectioned view of a prosthesis in which its lining is deflated, according to one embodiment of the invention.
**Fig. 6b** is a sectioned view of a prosthesis in which its lining is inflated, according to one embodiment of the invention.

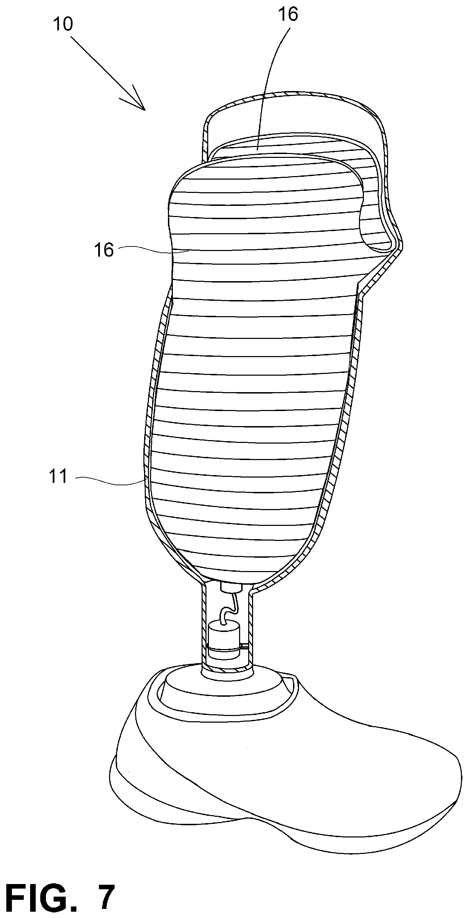

**Fig. 7** schematically illustrates a prosthesis not according to the invention.

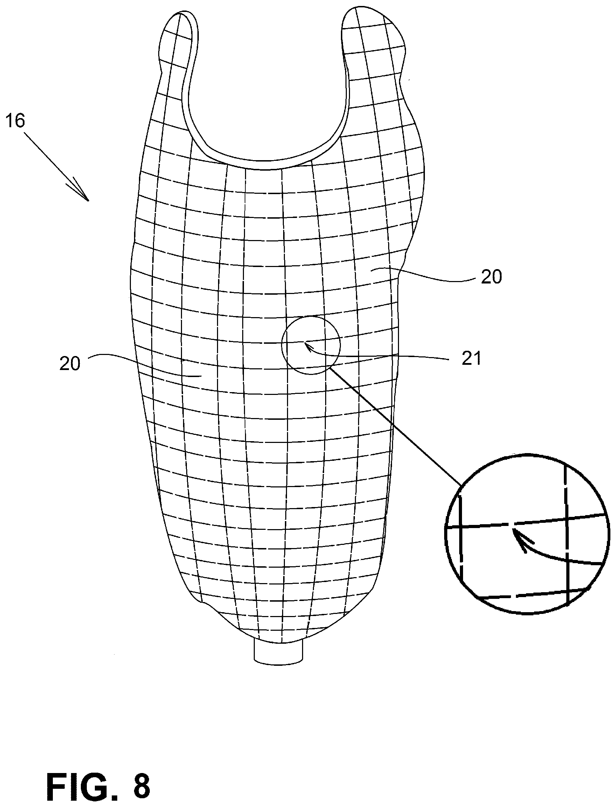

**Fig. 8** schematically illustrates a lining of a prosthesis not according to the invention.

It should be understood that the drawings are not necessarily drawn to scale.

### DESCRIPTION OF EMBODIMENTS

The present invention will be understood from the following detailed description of preferred embodiments ("best mode"), which are meant to be descriptive and not limiting. For the sake of brevity, some well-known features, methods, systems, procedures, components, circuits, and so on, are not described in detail.

**Fig. 4** schematically illustrates a prosthesis, according to one embodiment of the invention.

In this figure the socket 11 is longitudinally sectioned, for showing the parts of the prosthesis which are disposed inside the socket 11.

The prosthesis comprises an inflatable lining 16, inflatable by a pump assembly 19, and a pressure sensor (not shown) for measuring the air pressure inside the inflatable lining 16. Despite of the fact that the inlet from the pump assembly to the lining is drawn in Fig. 4 from the bottom of the lining, it should be noted that the inlet can be positioned elsewhere in the lining.

The pump assembly may be disposed in an unused space of the prosthesis, such as the prosthetic foot 12, and the joint pipe 13.

A communication assembly (not shown in this figure) disposed at the prosthesis has data connection with the pump assembly. The communication assembly communicates with the remote control 17 (preferably via wireless communication 18 such as Wi-Fi). The remote control may be in a form of a smartphone which executes a tailored application.

The user interface of the remote control 17 provides means through which a user can set the desired pressure inside the inflatable lining 16, to deflate (i.e., to release the air trapped inside the lining) the lining 16, and so on.

The prosthesis uses a controller (not illustrated in this figure), which instructs the pump assembly to inflate the inflatable lining 16 to a desired pressure. The desired pressure is set by a user via the remote control 17.

Alternatively, the user may order the pump assembly to inflate the lining, and when he feels that the air pressure is adequate, he may stop the inflating. The prosthesis may be retain this pressure until the user instructs the controller release the pressure (e.g., via a valve).

During the day the pressure inside the inflated lining may decrease due to leg contraction and air leakage. The controller may be set to re-inflate the inflatable lining 16 up to the pressure that has been set by the user.

The controller may reside in the prosthesis 10 or in the remote control 17. The controller may track the pressure inside the inflatable lining, and once it decreases, the controller can instruct the pump assembly to re-inflate the lining 16 to the set up pressure.

**Fig. 5** is a block diagram schematically illustrating the dataflow in a prosthesis, according to one embodiment of the invention.

For example, the user instructs the pump assembly to inflate the inflatable lining, and then to keep this pressure inside the lining.

The user sets the operation mode by the user interface, which sends the setup information to the controller via wireless communication means.

The controller instructs the pump assembly to inflate the inflatable lining, and while the lining is inflated, the pressure inside the lining is measured.

From the moment the user has instructed (via the user interface) the pump assembly to stop inflating, the controller acquires the pressure inside the lining (by reading the pressure sensor). From this stage the controller alternately or continuously acquires the air pressure inside the inflated lining (by reading the pressure sensor), and if the pressure has been changed from the one that has been set, e.g., by 3%, the controller instructs the pump assembly to inflate / deflate the lining to the pressure that has been set. The deflation is carried out by releasing air from the lining (e.g., by a controllable valve).

As mentioned above, the controller may reside at the prosthesis side, or at the remote control side.

**Fig. 6a** is a sectioned view of a prosthesis in which its lining is deflated, according to one embodiment of the invention.

**Fig. 6b** is a sectioned view of a prosthesis in which its lining is inflated, according to one embodiment of the invention.

The lining comprises inflatable cells 20, connected each other by air passages. Thus, when a residual limb of a user is disposed inside the lining, as the lining is inflated its grip to the user's limb increases.

The user may stop the inflating operation by the remote control.

When the user feels the grip of the lining is getting loose, he may increase the air pressure inside the lining by the remote control device. Additionally or alternatively, the controller may increase the air pressure inside the inflatable lining.

The cells inside the lining prevent from the air inside the lining to move from a side of the lining in which the pressure is "high" to another side of the lining in which the pressure is "low". Lack of cells will cause the leg not to be fixed inside the pillow, so the user's posture will not be stable. The cells provide to the lining softness along with posture.

For allowing inflating all the cells simultaneously, the lining's cells are interconnected.

**Fig. 7** schematically illustrates a prosthesis not according to the invention.

In this figure the socket 11 is sectioned in order to reveal the inner structure.

In addition, the lining 16 is shown in the figures herein as transparent, in order to show its cells.

As illustrated, the lining 16 comprises latitudinal cells.

Referring back to Fig. 4, the lining 16 comprises longitudinal cells 20. , There is an air passage (not illustrated in these figures) between each of the adjacent cells for allowing inflating and deflating the cells simultaneously by a single pump.

**Fig. 8** schematically illustrates a lining of a prosthesis not according to the invention.

The lining is illustrated from a front view.

Each inflatable cell 20 of the lining comprises an air passage 21 with its adjacent cells.

In this example, the inflatable cells 20 are in a crisscross order.

It should be noted that the prosthesis may be oriented to above-the-knee as well as to under-the-knee, and actually to any limb including hand.

In the figures and/or description herein, the following reference numerals (Reference Signs List) have been mentioned:
- numeral 10 denotes a prosthesis;
- numeral 11 denotes a socket of a prosthesis;
- numeral 12 denotes a prosthetic foot of a prosthesis;
- numeral 13 denotes a joint pipe connecting a socket 11 with a prosthetic foot 12;
- numeral 14 denotes a foot stump;
- numeral 15 denotes a sock;
- numeral 16 denotes a lining;
- numeral 17 denotes a remote control device;
- numeral 18 denotes wireless communication;
- numeral 19 denotes a pump assembly;
- numeral 20 denotes an inflatable cell; and
- numeral 21 denotes an air passage in a cell of a lining.

In the description herein, the following references have been mentioned: US5405405A.

The foregoing description and illustrations of the embodiments of the invention has been presented for the purposes of illustration. It is not intended to be exhaustive or to limit the invention to the above description in any form. The scope of the present invention is solely defined by the appended claims.

Any term that has been defined above and used in the claims, should be interpreted according to this definition.

The reference numbers in the claims are not a part of the claims, but rather used for facilitating the reading thereof. These reference numbers should not be interpreted as limiting the claims in any form.

## Claims

1. A limb prosthesis (10) comprising:
means for changing an inner size of a socket (11) of said limb prosthesis (10), said means comprising:
an inner inflatable lining (16) and a pump assembly (19) for inflating and deflating said inflatable lining (16),
wherein said inner inflatable lining (16) comprises only longitudinal inflatable cells (20) adjoining each other,
**characterized in that** said means for changing an inner size of the socket (11) of said limb prosthesis (10) further comprises a remote control (17) in data communication with said pump assembly (19), said remote control (17) configured to instruct said pump assembly (19) to inflate or deflate said lining (16) to a desired air pressure;
and **in that** each inner inflatable cell (20) comprises
an inwardly-facing surface having a convex curvature, each cell (20) being configured to be inflated to said desired air pressure, thereby generating a gap between each two adjoining cells (20) and a stump placed in said prosthesis.

2. The limb prosthesis according to claim 1, further comprising a pressure sensor and a controllable valve, for allowing inflating and deflating said lining (16) to said desired pressure.

3. The limb prosthesis according to claim 1, further comprising a circuitry adapted to retain constant pressure in said lining.

4. The limb prosthesis according to claim 1, wherein said remote control (17) is a smartphone.

5. The limb prosthesis according to claim 1, wherein said data communication is wired.

6. The limb prosthesis according to claim 1, wherein said data communication is wireless.

7. The limb prosthesis according to claim 1, further comprising means for manually deflating said lining, thereby allowing deflating said lining in the case of an operational fault.

8. The limb prosthesis according to claim 1, wherein said means for changing an inner size of a socket of said limb prosthesis by a remote control are pneumatic.

## Patentansprüche

1. Gliedmaßenprothese (10), umfassend:
Mittel zum Verändern einer Innenabmessung einer Aufnahme (11) der Gliedmaßenprothese (10), wobei die genannten Mittel Folgendes umfassen:
eine innere aufblasbare Auskleidung (16) und eine Pumpenanordnung (19) zum Aufblasen und Entleeren der aufblasbaren Auskleidung (16),
wobei die innere aufblasbare Auskleidung (16) nur längsverlaufende aufblasbare Zellen (20) umfasst, die aneinander angrenzen,
**dadurch gekennzeichnet, dass** die Mittel zum Verändern einer Innenabmessung der Aufnahme (11) der Gliedmaßenprothese (10) ferner eine Fernbedienung (17) in Datenkommunikation mit der Pumpenanordnung (19) umfassen, wobei die Fernbedienung (17) dazu konfiguriert ist, die Pumpenanordnung (19) anzuweisen, die Auskleidung (16) auf einen gewünschten Luftdruck aufzublasen oder zu entleeren;
und dass jede innere aufblasbare Zelle (20) eine nach innen gewandte Oberfläche mit einer konvexen Krümmung umfasst, wobei jede Zelle (20) dazu konfiguriert ist , auf den gewünschten Luftdruck aufgeblasen zu werden, wodurch ein Spalt zwischen jeweils zwei benachbarten Zellen (20) und einem in der Prothese platzierten Stumpf gebildet wird.

2. Gliedmaßenprothese nach Anspruch 1, ferner umfassend einen Drucksensor und ein steuerbares Ventil, um das Aufblasen und Entleeren der Auskleidung (16) auf den gewünschten Druck zu ermöglichen.

3. Gliedmaßenprothese nach Anspruch 1, ferner umfassend eine Schaltung, die dazu angepasst ist, einen konstanten Druck in der Auskleidung zu halten.

4. Gliedmaßenprothese nach Anspruch 1, wobei die Fernbedienung (17) ein Smartphone ist.

5. Gliedmaßenprothese nach Anspruch 1, wobei die Datenkommunikation drahtgebunden ist.

6. Gliedmaßenprothese nach Anspruch 1, wobei die Datenkommunikation drahtlos ist.

7. Gliedmaßenprothese nach Anspruch 1, ferner umfassend Mittel zum manuellen Entleeren der Auskleidung, wodurch das Entleeren der Auskleidung im Falle einer Betriebsstörung ermöglicht wird.

8. Gliedmaßenprothese nach Anspruch 1, wobei die Mittel zum Verändern einer Innenabmessung einer Aufnahme der Gliedmaßenprothese durch eine Fernbedienung pneumatisch sind.

## Revendications

1. Prothèse de membre (10) comprenant :
un moyen pour changer une taille interne d'une emboîture (11) de ladite prothèse de membre (10), ledit moyen comprenant :
une doublure gonflable interne (16) et un ensemble pompe (19) pour gonfler et dégonfler ladite doublure gonflable (16),
ladite doublure gonflable interne (16) comprenant uniquement des cellules gonflables longitudinales (20) adjacentes les unes aux autres,
**caractérisée en ce que** ledit moyen pour changer une taille intérieure de l'emboîture (11) de ladite prothèse de membre (10) comprend en outre une télécommande (17) en communication de données avec ledit ensemble pompe (19), ladite télécommande (17) étant configurée pour ordonner audit ensemble pompe (19) de gonfler ou de dégonfler ladite doublure (16) à une pression d'air souhaitée ;
et **en ce que** chaque cellule gonflable interne (20) comprend une surface tournée vers l'intérieur présentant une courbure convexe, chaque cellule (20) étant conçue pour être gonflée à ladite pression d'air souhaitée, générant ainsi un espace entre toutes les deux cellules adjacentes (20) et un moignon placé dans ladite prothèse.

2. Prothèse de membre selon la revendication 1, comprenant en outre un capteur de pression et une valve contrôlable, pour permettre le gonflage et le dégonflage de ladite doublure (16) à ladite pression souhaitée.

3. Prothèse de membre selon la revendication 1, comprenant en outre un circuit conçu pour maintenir une pression constante dans ladite doublure.

4. Prothèse de membre selon la revendication 1, ladite télécommande (17) étant un smartphone.

5. Prothèse de membre selon la revendication 1, ladite communication de données étant câblée.

6. Prothèse de membre selon la revendication 1, ladite communication de données étant sans fil.

7. Prothèse de membre selon la revendication 1, comprenant en outre un moyen pour dégonfler manuellement ladite doublure, permettant ainsi de dégonfler ladite doublure en cas de défaut de fonctionnement.

8. Prothèse de membre selon la revendication 1, ledit moyen pour changer une taille interne d'une emboîture de ladite prothèse de membre par une télécommande étant pneumatique.
